# EUROPÄISCHE PATENTSCHRIFT

(11) **EP 1 806 989 B1**
(45) Veröffentlichungstag und Bekanntmachung des Hinweises auf die Patenterteilung: **15.05.2013**
(21) Anmeldenummer: 05800396.3
(22) Anmeldetag: 27.10.2005
(51) Int. Cl.: A44B 18/00, A61F 13/62

(54) **SCHLAUFENBILDENDES VLIESSTOFFMATERIAL FÜR EIN MECHANISCHES VERSCHLUSSMITTEL**
LOOP-FORMING NONWOVEN MATERIAL FOR A MECHANICAL CLOSURE ELEMENT
MATIERE NON TISSEE FORMANT DES BOUCLES COMME ELEMENT D'OBTURATION MECANIQUE

(30) Priorität: 03.11.2004 DE 102004053469
(43) Veröffentlichungstag der Anmeldung: 18.07.2007
(73) Patentinhaber: Paul Hartmann AG, 89522 Heidenheim (DE)
(72) Erfinder: STUPPERICH, Hans-Peter, 89552 Heidenheim (DE); HORNUNG, Fridmann, 73466 Lauchheim (DE); KESSELMEIER, Rüdiger, 89542 Herbrechtingen (DE)
(74) Vertreter: Friz, Oliver
(86) Internationale Anmeldenummer: PCT/EP2005/011492
(87) Internationale Veröffentlichungsnummer: WO 2006/048173

(56) Entgegenhaltungen:
- US-A1- 2004 163 221

## Beschreibung

Die Erfindung betrifft ein schlaufenbildendes Vliesstoffmaterial für ein mechanisches Verschlussmittel, insbesondere bei wegwerfbaren Hygieneartikeln, wie Windeln, Gürtelwindeln, Inkontinenzartikeln und -vorlagen, wobei eine erste Oberseite des Vliesstoffmaterials erste größere ungebundene Bereiche umfasst, die voneinander beabstandet inselartig angeordnet sind.

Bei der Herstellung von mechanisch wirkenden Verschlussmitteln, die häufig als Haken/Schlaufen-Verschlussmittel oder Klettverschlussmittel bezeichnet werden, soll einerseits eine gute auf Hintergriffen oder Verhakungen beruhende Verschlusswirkung der zusammenwirkenden Verschlusselemente erreicht werden, die sich nicht unbeabsichtigt lösen. Andererseits sollen diese Verschlussmittel aber auch in beabsichtigter Weise gelöst werden können, und zwar ohne dass Materialien der Verschlusselemente abgelöst oder herausgerissen werden. Um letzteres zu vermeiden, werden bei der schlaufenbildenden Komponente des mechanischen Verschlussmittels entweder textile Bindungen vorgesehen oder - wenn mit Vliesstoffen gearbeitet wird - wird für eine hinreichende Verfestigung oder Bindung der Fasern des Vlieses gesorgt. Mit zunehmender Vliesverfestigung geht aber eine Abnahme der hiermit erzielbaren Haftkraft des mechanischen Verschlusssystems einher.

Mit EP 0 870 081 B1, die ein schlaufenbildendes Vliesstoffmaterial nach dem Oberbegriff des Anspruchs 1 beschreibt, wurde bereits vorgeschlagen, ausgehend von einer vorzugsweise durchgehend ungebundenen Faservliesbahn inselartige ungebundene Bereiche dadurch vorzusehen, dass zwischen diesen ungebundenen Bereichen durch Kalanderprägen eine durchgehende Bindung des Vlieses vorgesehen wird. Die durch Kalanderprägung verfestigten Gebiete zwischen den inselartigen ungebundenen Bereichen stehen dann aber zur Ausbildung von Haftbindungen praktisch nicht mehr zur Verfügung.

EP 1 209 271 A1 offenbart ein loftiges Vliesstoffmaterial, welches voneinander isolierte liniensegmentartige gebundene Bereiche aufweist und Vliesstoffmaterialien, bei denen ein waffelförmiges Muster von Prägepunkten vorgesehen ist. WO 95/33390 zeigt ein Vlies-/Folienlaminat, bei dem eine vorverfestigte Faservliesbahn über ein Raster diskreter Fügepunkte an eine elastomere Filmschicht angefügt ist.

EP 1 048 236 A2 schlägt vor, auf einer wärmeschmelzbaren Basisschicht wärmeschmelzbare kontinuierliche Fasern in paralleler Anordnung zueinander abzulegen und mit der Basisschicht durch quer zu der Faserrichtung erstreckte gerade Fügelinien zu verbinden. Auf diese Weise werden zwischen den Fügelinien Schlaufen gebildet, die mit einer hakenbildenden Komponente eines Verschlussmittels zusammenwirken können.

Der vorliegenden Erfindung liegt die Aufgabe zugrunde, unter Berücksichtigung des eingangs erwähnten Zielkonflikts bei der Ausbildung einer schlaufenbildenden Komponente eines mechanischen Verschlussmittels ein im Hinblick auf eine sichere Schließfunktion und eine geringe Zerstörungsanfälligkeit verbessertes Vliesstoffmaterial zu schaffen.

Diese Aufgabe wird durch ein schlaufenbildendes Vliesstoffmaterial mit den Werkmalen des Anspruchs 1 gelöst.

Die größeren ungebundenen Bereiche stehen zur Ausbildung einer mechanisch wirkenden Verschlussfunktion primär zur Verfügung. Sie sind durch eine gebundene Kontur begrenzt oder definiert, die insbesondere durch linienförmige Prägung, insbesondere eine Thermoprägung, die insbesondere durch Ultraschallschweißen oder Kalanderprägung erzeugt ist, gebildet sein kann, wobei diese Prägelinien durchgehend oder unterbrochen sein können, insbesondere auch punktiert oder strichpunktiert ausgebildet sein können. Außerhalb dieser gebundenen Kontur sind aber zwischen diesen ersten ungebundenen inselartigen Bereichen weitere kleinere ungebundene Bereiche oder Gebiete vorgesehen, welche die ersten größeren ungebundenen Bereiche voneinander trennen. Wenn in diesem Zusammenhang von zweiten kleineren ungebundenen Bereichen oder Gebieten die Rede ist, so soll hierunter verstanden werden, dass die inselartigen größeren Bereiche entweder derart nah beieinander oder aneinander angeordnet sind oder die zweiten kleineren ungebundenen Bereiche ihrerseits gebundene Teilbereiche umschließen, dass es nicht möglich ist, eine größtmögliche, noch einbeschriebene Kreisfläche der ersten größeren ungebundenen Bereiche in den zweiten ungebundenen Bereichen aufzunehmen oder einzulegen.

Mit dem erfindungsgemäßen Vliesstoffmaterial wird einerseits durch die ersten größeren ungebundenen inselartigen Bereiche ein insgesamt und auch jeweils für sich betrachtet ausladender und hinreichend großer Verankerungsbereich für die hakenbildende Komponente des mechanischen Verschlussmittels zur Verfügung gestellt. Die weiteren zweiten ungebundenen Bereiche zwischen den inselartigen ersten ungebundenen Bereichen tragen aber auch zu einer möglichst kontinuierlichen mechanischen Verbindung bei. Eine gleichwohl hinreichende Verfestigung des schlaufenbildenden Vliesstoffmaterials wird durch die die ersten ungebundenen Bereiche begrenzenden gebundenen Konturen erreicht und gegebenenfalls unterstützt durch weitere gebundene Teilbereiche, welche ihrerseits von den zweiten ungebundenen Bereichen umgeben zwischen den ersten größeren Bereichen angeordnet sind.

Im Hinblick auf eine gute Festigkeit und eine gute Einbindung der Fasern des Vliesstoffmaterials erweist es sich als vorteilhaft, wenn - wie bereits erwähnt - die zweiten kleineren ungebundenen Bereiche ihrerseits gebundene Teilbereiche aufweisen, insbesondere umschließen. Diese Teilbereiche, die ebenfalls durch Prägung, insbesondere Thermoprägung, gebildet werden können, haben an sich beliebige Form, wobei sich eine Ausbildung der Teilbereiche in Form verhältnismäßig kurzer und schmaler Segmente als vorteilhaft erweist, deren Längserstreckung etwa das Zweibis Zehnfache, insbesondere das Zwei- bis Achtfache ihrer Breite beträgt.

Des Weiteren erweist es sich im Hinblick auf eine gute Einbindung der Fasern in das Vliesstoffmaterial als vorteilhaft, wenn jede gerade Verbindungslinie zwischen den ersten größeren ungebundenen inselartigen Bereichen stets durch eine gebundene Kontur oder einen gebundenen Teilbereich, der zwischen den ersten größeren Bereichen angeordnet ist, verläuft.

Die gebundenen Konturen weisen vorteilhafterweise Prägelinien oder Prägelinienabschnitte einer Breite von 0,2 bis 1 mm, vorzugsweise von 0,2 bis 0,8 mm und weiter vorzugsweise von 0,3 bis 0,6 mm auf. Sie haben vorteilhafterweise eine Tiefe von 0,4 bis 1,5 mm, vorzugsweise von 0,4 bis 0,9 mm, weiter vorzugsweise von 0,4 bis 0,8 mm und weiter vorzugsweise von 0,5 bis 0,7 mm.

Es hat sich des Weiteren als vorteilhaft erwiesen, wenn die ersten größeren ungebundenen inselartigen Bereiche eine Abmessung, insbesondere einen Durchmesser eines in den Bereich einbeschriebenen Kreises, von vorzugsweise von 3 bis 10 mm, weiter vorzugsweise von 3 bis 8 mm und besonders bevorzugtermaßen von 4 bis 7 mm aufweisen.

Die ersten größeren ungebundenen inselartigen Bereiche haben vorzugsweise einen Flächenanteil von 10 - 45 %, insbesondere von 15 - 45 %, insbesondere von 20 - 40 % und weiter insbesondere von 30 - 40 % der Gesamtfläche der ersten Oberseite. Es wurde erfindungsgemäß erkannt, dass die ersten größeren ungebundenen inselartigen Bereiche in der Summe auch einen verhältnismäßig moderaten Anteil der gesamten Oberfläche ausmachen können, da die Verschlussfunktion zusätzlich von den zwar kleineren ungebundenen Bereichen oder Gebieten zwischen den ersten größeren ungebundenen Bereichen unterstützt wird.

Die ersten größeren ungebundenen inselartigen Bereiche haben keine notwendigerweise bestimmte Form, es erweist sich jedoch als vorteilhaft, wenn sie kreisförmig oder oval oder dreieckig oder vieleckig, vorzugsweise sechseckig oder achteckig, ausgebildet sind. Durch diese bevorzugten Strukturen lässt sich ein flächenmäßig ausladender Ankerbereich für die hakenbildende Komponente des Verschlussmittels darstellen. Es zeigt sich nämlich, dass die Verschlussfunktion durch verhältnismäßig große inselartige Bereiche, deren Abmessung vorzugsweise in allen Richtungen gleich ist, so wie dies beim Kreis oder regelmäßigen Vieleck, insbesondere Sechseck der Fall ist, besonders effektiv ist.

Im Hinblick auf eine Anordnung der ersten inselartigen Bereiche hat es sich als besonders zweckmäßig erwiesen, wenn diese wenigstens 1 mm, vorzugsweise wenigstens 1,5 mm, insbesondere wenigstens 2 mm und weiter insbesondere wenigstens 2,5 mm und besonders bevorzugtermaßen wenigstens 3 mm voneinander beabstandet sind. Sie sind jedoch vorzugsweise höchstens 10 mm, insbesondere höchstens 5 mm voneinander beabstandet.

Eine gute und ausreichende Stabilität des Vliesmaterials, d. h. eine gute Einbindung der Fasern und damit eine geringe Zerstörungsanfälligkeit des Vliesstoffmaterials lässt sich erreichen, wenn der gesamte Flächenanteil gebundener Bereiche vorzugsweise 15 bis 40 %, weiter vorzugsweise 15 bis 30 %, insbesondere 15 bis 25 % und in besonders vorteilhafter Weise 19 bis 22 % der Gesamtfläche der ersten Oberfläche beträgt. Wenn hier von gebundenen Bereichen die Rede ist, so werden hierunter diejenigen gebundenen Konturen, welche die ersten inselartigen Bereiche begrenzen und auch die gegebenenfalls zusätzlich vorhandenen gebundenen Teilbereiche zwischen den ersten inselartigen Bereichen verstanden.

Das erfindungsgemäße Vliesstoffmaterial kann bevorzugtermaßen ein Spinnvlies oder ein Kardenvlies oder ein Meltblownvlies oder ein wasservernadeltes Vlies umfassen. Es kann sich in vorteilhafter Weise auch um ein Vlieslaminat handeln, welches aus mehreren Vliesschichten besteht. Nach einer bevorzugten Ausführungsform der Erfindung umfasst das Material ein Spinnvlies und ein Kardenvlies, die miteinander verbunden sind. Solchenfalls bildet das Kardenvlies in bevorzugter Weise die erste Oberseite, welche eine Landezone für eine hakenbildende Komponente eines mechanischen Verschlussmittels bildet.

Wenn das erfindungsgemäße schlaufenbildende Vliesstoffmaterial aus mehreren Vliesschichten besteht, es sich also um ein Vlieslaminat handelt, so erweist es sich als vorteilhaft, wenn diese Schichten durch Aufbringung der die ersten größeren ungebundenen inselartigen Bereiche begrenzenden gebundenen Konturen miteinander verbunden werden. Dies kann beispielsweise durch Thermoprägung, und zwar insbesondere durch Kalanderprägen oder Ultraschallschweißen erreicht werden. Bei der Ausbildung des erfindungsgemäßen Vliesstoffmaterials als Vliesstofflaminat kann, um die Festigkeit insgesamt zu erhöhen, eine von der ersten Oberseite abgewandte Vliesschicht durch ein weiteres, insbesondere durch Thermoprägung erzeugtes Bindemuster verfestigt sein. Diese weitere Vliesschicht bildet also nicht die Landezone für eine hakenbildende Komponente des Verschlussmittels, sondern sie ist auf der abgewandten Seite vorgesehen. Das Bindemuster wurde vorzugsweise bei der vorherigen Fertigung der weiteren Vliesschicht zu deren Vorverfestigung angebracht.

Das Flächengewicht eines erfindungsgemäßen Vliesmaterials beträgt bevorzugtermaßen 15 bis 120 g/m², insbesondere von 20 bis 90 g/m², insbesondere von 30 bis 80 g/m², insbesondere von 40 bis 70 g/m² und weiter insbesondere von 50 bis 65 g/m².

Das Flächengewicht derjenigen Vliesschicht, welche die erste Oberseite bildet und die ersten ungebundenen inselartigen Bereiche, die gebundenen Konturen und die zweiten ungebundenen Bereiche aufweist, beträgt vorzugsweise 10 bis 60 g/m², insbesondere von 10 bis 40 g/m², insbesondere von 15 bis 35 g/m² und weiter insbesondere von 20 bis 35 g/m².

Mit den vorerwähnten Flächengewichten in Verbindung mit der beanspruchten Ausgestaltung der ersten Oberseite lassen sich Vliesstoffmaterialien herstellen, die nicht nur eine gute Verschlussfunktion im Zusammenwirken mit einer hakenbildenden Komponente eines Verschlussmittels gewährleisten, sondern die zudem gute mechanische Eigenschaften insbesondere im Hinblick auf eine angenehme Biegsamkeit aufweisen und zudem auch tragende Funktion übernehmen können. Dies bedeutet, dass diese Vliesstoffmaterialien insbesondere als Gürtel bei absorbierenden Hygieneartikeln einsetzbar sind.

Im Hinblick auf die Ausbildung des Vliesstoffmaterials erweist es sich als vorteilhaft und zweckmäßig, wenn diejenige Vliesschicht, welche die erste Oberseite bildet, Fasern einer Stärke von 1 bis 10 dtex, vorzugsweise von 2 bis 8 dtex und weiter bevorzugtermaßen von 3 bis 6 dtex umfasst oder aus solchen Fasern besteht. Des Weiteren erweist es sich als vorteilhaft, wenn diejenige Vliesschicht, welche die erste Oberseite bildet, hydrophile Fasern umfasst oder aus hydrophilen Fasern besteht.

Wenn das erfindungsgemäße Vliesstoffmaterial aus mehreren Vliesschichten besteht, so kann es eine Vliesschicht als Träger umfassen, die ein Flächengewicht von 10 bis 100 g/m², vorzugsweise von 15 bis 60 g/m² und weiter vorzugsweise von 30 bis 40 g/m² aufweist. Diese Trägerschicht ist dann von der ersten Oberseite abgewandt angeordnet. Sie umfasst vorzugsweise Fasern einer Stärke von 1 bis 6 dtex, vorzugsweise von 1 bis 4 dtex und weiter vorzugsweise von 2 bis 4 dtex.

In besonders vorteilhafter Weiterbildung der Erfindung ist das Vliesstoffmaterial atmungsaktiv, das heißt es lässt Wasser passieren, und /oder luftdurchlässig. Hierdurch wird der Tragekomfort verbessert, insbesondere dann, wenn das Vliesstoffmaterial als Gürtel bei absorbierenden Hygieneartikeln eingesetzt wird.

Wie bereits angedeutet, erweist es sich als vorteilhaft, wenn das erfindungsgemäße Vliesstoffmaterial eine Steifigkeit von < 0,80 N, insbesondere von < 0,60 N, insbesondere von < 0,40 N, insbesondere von < 0,30 N, insbesondere von < 0,25 N, insbesondere von < 0,2 N, insbesondere < 0,18 N, und weiter insbesondere < 0,16 N, jedoch von wenigstens 0,05 N aufweist. Diese Steifigkeit wird bestimmt nach der in EP 0 699 066 B1 beschriebenen Prüfmethode der modifizierten Version des Tests ASTM D 4032-82 CIRCULAR BEND, so dass zu Zwecken der Offenbarung hierauf ausdrücklich Bezug genommen wird und der diesbezügliche Inhalt von EP 0 699 066 B1 in die vorliegende Anmeldung mit einbezogen wird. Ergänzend hierzu wird diese Prüfmethode dahingehend weiter spezifiziert, dass bei der Prüfanordnung die erste Oberseite des Vliesstofflaminates unten zu liegen kommt, das heißt diejenige Oberfläche bildet, die dem Stempel der Prüfapparatur abgewandt ist.

Gegenstand der vorliegenden Erfindung ist weiterhin ein Hygieneartikel zum einmaligen Gebrauch, insbesondere eine Windel, eine Gürtelwindel, eine Inkontinenzwindel oder eine Inkontinenzvorlage, die gekennzeichnet ist durch ein mechanisches Verschlussmittel, welches ein schlaufenbildendes Vliesstoffmaterial nach einem oder mehreren der Ansprüche 1 bis 24 umfasst bzw. hieraus gebildet ist.

Bei einem derartigen Hygieneartikel kann die weibliche Klettverschlusskomponente einer an sich bekannten Wegwerfwindel der herkömmlichen Art(siehe WO95/33390) dieses erfindungsgemäße Vliesstoffmaterial umfassen oder daraus bestehen.

Ein derartiger Hygieneartikel kann aber auch einen in Hüftumfangsrichtung eines Benutzers erstreckten und auf sich selbst schließbaren Gürtel umfassen. Dieser Gürtel bildet solchenfalls eine geschlossene Hüftöffnung, und ein die absorbierenden Komponenten des Hygieneartikels aufweisender Hauptteil wird beim Anlegen des Artikels zwischen den Beinen eines Benutzers hervorgeholt und an dem zuvor geschlossenen Gürtel vorzugsweise lösbar festgelegt.

In weiterer Ausbildung der Erfindung von besonderer Bedeutung ist der Gürtel aus einem vorzugsweise einstückigen Materialabschnitt gebildet, welcher Materialabschnitt ein Vliesstoffmaterial nach einem oder mehreren der Ansprüche 1 bis 24 umfasst oder hieraus gebildet ist.

Es erweist sich insofern als vorteilhaft, wenn der Gürtel eine Erstreckung in Hüftumfangsrichtung von 25 bis 200 cm, insbesondere von 40 bis 180 cm, insbesondere von 60 bis 180 cm, insbesondere von 100 bis 180 cm und weiter von 120 bis 180 cm aufweist.

Des Weiteren erweist es sich als vorteilhaft, dass der Gürtel oder die schlaufenbildende Komponente, die von einem erfindungsgemäßen Vliesstoffmaterial gebildet ist, eine Erstreckung in Längsrichtung des Hygieneartikels von 3 bis 20 cm, insbesondere von 3 bis 15 cm, insbesondere von 4 bis 10 cm und weiter insbesondere von 4 bis 6 cm aufweist.

Es wird weiter Schutz beansprucht für einen Hygieneartikel, bei dem das schlaufenbildende Vliesstoffmaterial mit einer hakenbildenden Komponente eines mechanischen Verschlussmittels zum Schließen des Hygieneartikels derart zusammenwirkt, dass eine Haftkraft als Scherkraft von wenigstens 5 N/25 mm und höchstens 80 N/25 mm erreicht wird. Bevorzugte Bereiche ergeben sich aus den Ansprüchen.

In diesem Zusammenhang wird auch darauf hingewiesen, dass Schutz begehrt wird für ein mechanisches Verschlussmittel, zur Verwendung bei absorbierenden Hygieneartikeln, dessen schlaufenbildende Komponente ein erfindungsgemäßes Vliesstoffmaterial umfasst bzw. aus einem solchen erfindungsgemäßen Vliesstoffmaterial besteht.

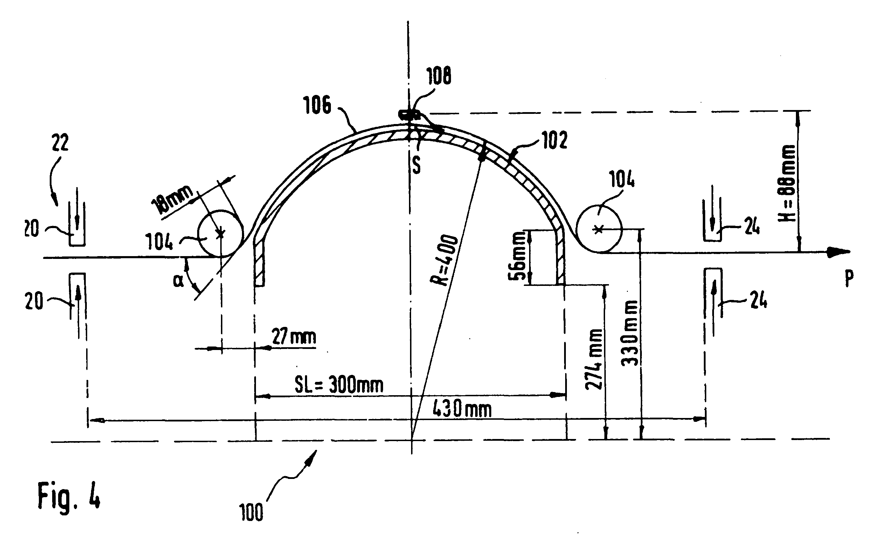

Nachfolgend wird eine Prüfmethode für die Ermittlung der Verschlusskräfte bei Scherbeanspruchung angegeben. Für die Durchführung der Prüfmethode kann ein Zugprüfgerät Typ Z010 / TN 2S, Messdose 100 N, erhältlich bei der Firma Zwick GmbH & Co KG, Ulm, Deutschland, mit einer Klemmbackenbreite zum Einspannen des Prüflings von 60 mm verwendet werden. Bei der Durchführung der Prüfmethode wird das zu prüfende Verschlusssystem mit einer schlaufenbildenden Komponente und einer darauf haftenden hakenbildenden Komponente über eine gekrümmte Fläche gelegt, welche eine Rundung des Bauchbereichs eines Benutzers simulieren soll (siehe Figur 4). Zur Verbindung der Verschlussmittel mit den Klemmbacken des Zugprüfgeräts wird ein biegsames Substrat, beispielsweise ein einseitig klebendes Klebeband einer bevorzugten Breite von 25 mm, das unter der Bezeichnung STA 306 bei der Firma 3M Deutschland GmbH ansässig in Neuss erhältlich ist, verwendet. Das Klebeband ist aus Polypropylen, seine Oberfläche ist beschichtet durch ein urethanmodifiziertes Siliconpolymer. Das Flächengewicht des Haftklebstoffauftrags beträgt 23 g/m². Der Der über die gekrümmte Fläche gelegte Prüfling, bestehend aus aufeinander haftenden flächenhaften Abschnitten der Verschlussmittel, wird durch Verwendung des Zugprüfgeräts auf Zug beansprucht, woraus sich eine scherende Beanspruchung der aneinander haftenden flächenhaften Abschnitte ergibt.

### Probenvorbereitung:

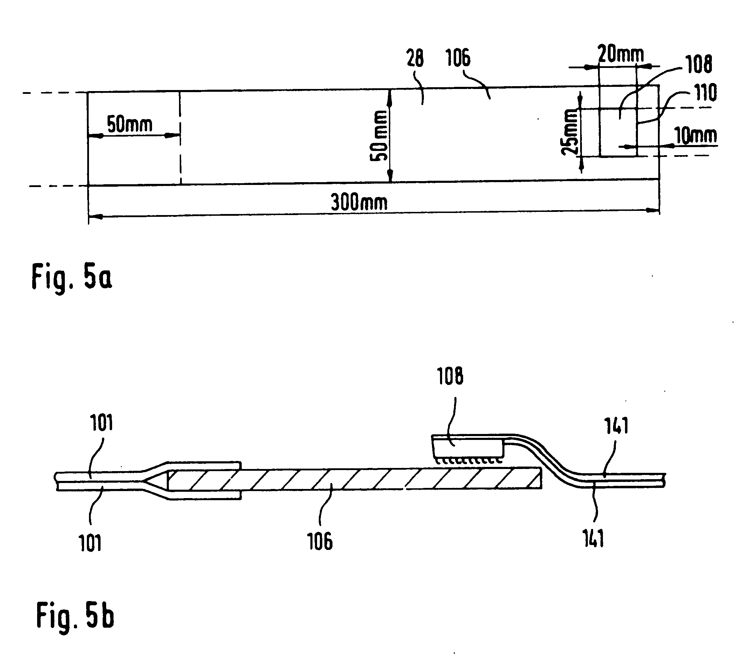

Die zu verwendenden mechanischen Verschlussmittel, also das schlaufenbildende Vliesstoffmaterial 106 und eine hakenbildende Komponente 108 des Verschlusssystems, werden über 24 h bei 23° C und 50 % relativer Luftfeuchte konditioniert. Es werden Prüflinge einer Größe von 50 x 300 mm aus dem schlaufenbildenden Vliesstoffmaterial ausgestanzt und sandwichartig mittig zwischen die Enden zweier einseitiger Klebebänder 101 einer Breite von 25 mm, die gegeneinander geklebt sind, angeordnet bzw. fixiert, so dass sich ein Überstand des Vliesstoffmaterials von 50 x 250 mm ergibt (siehe Figuren 5a, 5b).

Desgleichen wird von der hakenbildenden Komponente 108 des Verschlussmittels ein flächenhafter Abschnitt von 25 x 20 mm ausgestanzt und durch zwei mit ihren Klebeflächen gegeneinander geklebte einseitige Klebebänder 141 derart fixiert, dass das oberer Klebeband die Rückseite des flächenhaften Abschnittes überfängt und das untere Klebeband bündig an den flächenhaften Abschnitt angrenzt (siehe Figuren 5a und 5b).

Der flächenhafte Abschnitt der hakenbildenden Komponente 108 wird nun auf das schlaufenbildende Vliesstoffmaterial 106 aufgelegt, wobei der Abstand von der Längsendkante des Vliesstoffmaterials 10 mm und von den seitlichen Längsrändern je 12,5 mm betragen soll (s. Figur 5a).

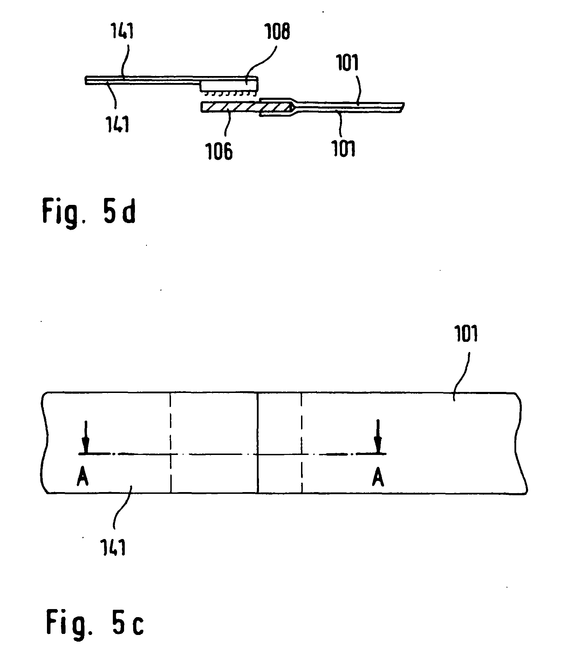

Wenn das zur Verfügung stehende schlaufenbildende Vliesstoffmaterial 106 von vornherein eine kleinere Abmessung aufweist, so dass keine Bereitstellung eines 50 mm x 300 mm größen Prüfling möglich ist, wird die Größe des Prüflings mit 25 mm x 30 mm gewählt und dieser Abschnitt sandwichartig mittig zwischen die Enden zweier einseitiger oben näher gekennzeichneter Klebebänder 101 einer Breite von 25 mm, die gegeneinander geklebt sind, angeordnet bzw. fixiert, so dass sich ein Überstand des Vliesstoffmaterials von 25 x 20 mm ergibt (siehe Figuren 5c, 5d). Solchenfalls werden die so vorbereiteten Abschnitte der Haken bildende Komponente 108 und des schlaufenbildenden Vliesmaterials 106 vollflächig übereinander gelegt (Figuren 5c, 5d).

Die so oder auf die zuvor beschriebene Art aufeinander gelegten flächenhaften Abschnitte werden durch viermaliges Anrollen mit einer 50 mm breiten und im Durchmesser 100 mm starken Rolle mit glatter Oberfläche und einem Rollengewicht von 5 kg miteinander verbunden, wobei die Anrollgeschwindigkeit 20-100 mm/sec beträgt.

### Prüfverfahren:

Das wie vorstehend beschrieben verlängerte schlaufenbildende Vliesstoffmaterial wird in die untere Klemmbacke des Zugprüfgeräts mittig zentriert eingespannt, und das gegenüberliegende Ende der wie vorstehend beschrieben verlängerten hakenbildenden Komponente wird in die bewegliche obere Klemmbacke des Zugprüfgeräts ebenfalls zentriert eingespannt.

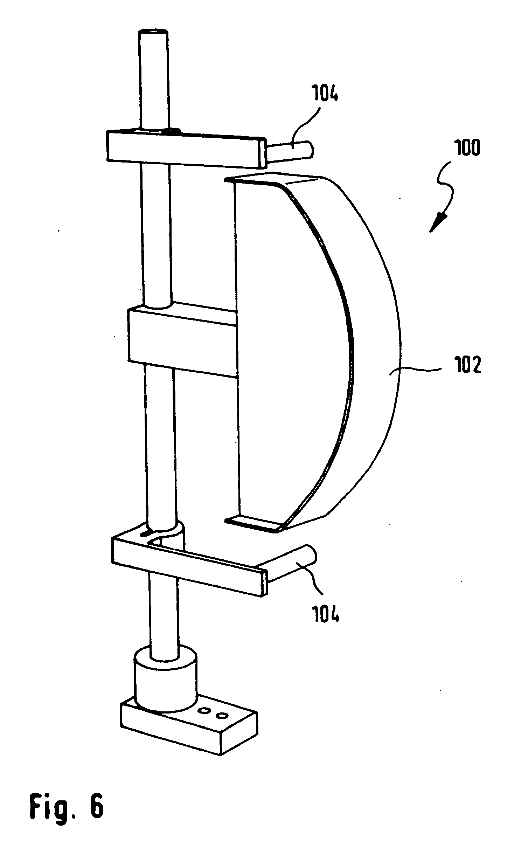

Der so eingespannte Prüfling wird über die aus Figur 4, 6 ersichtliche Vorrichtung 100, welche den Bauch- oder Hüftbereich eines Benutzers simulieren soll, gelegt. Diese Vorrichtung 100 ist in Figur 6 perspektiv dargestellt. Man erkennt eine bogenförmig gekrümmte Fläche 102 aus poliertem Stahl mit einer Rautiefe von 5 bis 25 µm und mit einem Krümmungsradius R von zumindest abschnittsweise 400 mm und einer Sehnenlänge SL von 300 mm. Des Weiteren sind oberhalb und unterhalb der gekrümmten Fläche 102 Umlenkrollen 104 mit einem Durchmesser von 18 mm vorgesehen, welche den über die gekrümmte Fläche gelegten Prüfling in die vertikale Richtung um H = 88 mm umlenken, wo er dann mit Klemmen 20, 24 des nicht dargestellten Zugprüfgeräts verbunden wird. Die Umlenkung erfolgt um einen Winkel α von 60°. Hierdurch wird der Abzugswinkel im Wesentlichen tangential zu der gekrümmten Fläche und konstant gehalten. Die aufeinander gelegten flächenhaften Abschnitte 106, 108 der Komponenten des Verschlussmittels werden in Bezug auf die gekrümmte Fläche 102 so positioniert, dass die hakenbildende Komponente mittig zentriert im Scheitelpunkt S der gekrümmten Fläche 102 zu liegen kommt.

Es wird dann die bewegliche Klemme 24, mit der die hakenbildende Komponente verbunden ist, mit der nachfolgend angegebenen Prüfgeschwindigkeit in Pfeilrichtung P bewegt, und es wird währenddessen die dabei auftretende Zugkraft zwischen den Klemmen ermittelt. Die Prüfparameter sind:
- Prüfgeschwindigkeit: 300 mm/min
- Einspannlänge des Prüflings: 430 mm (s. Figur 4)
- Messweg: Strecke bis zur Ablösung der Verschlussmittelkomponente n voneinander
- Vorkraft: 0,2 N
- Prüfanzahl: n ≥ 6.

Die Auswertung erfolgt dergestalt, dass die bis zum voneinander Ablösen der Verschlussmittel ermittelte Maximalkraft gerundet auf zwei Dezimalstellen in N (Newton) notiert wird und in Form eines Mittelwerts der n-Messungen und Angabe der Standardabweichung sowie eines Minimalwerts und eines Maximalwerts angegeben wird.

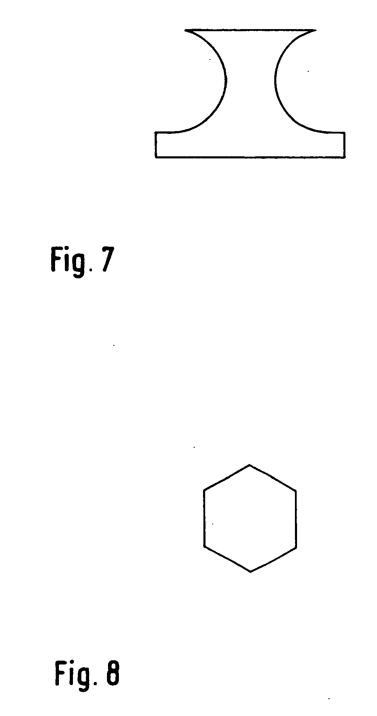

Eine hakenbildende Komponente, die zusammen mit dem erfindungsgemäßen Vliesstoffmaterial als schlaufenbildende Komponente eines mechanischen Verschlussmittels verwendet werden kann, ist beispielsweise unter dem Handelsnamen "Microplast" 42-288-HX200-PP3 von der Firma G. Binder GmbH & Co. KG Textil- und Kunststofftechnik, Holzgerlingen, Deutschland, zu erhalten. Darstellungen der Form der hakenbildenden Komponente sind in den Figuren 7 und 8 dargestellt. Die Haken haben eine pilzförmige Gestalt mit ungefähr hexagonaler Fläche des Kopfs. Es befinden sich etwa 288 solcher pilzförmiger Vorsprünge pro cm². Das Material besteht aus Polypropylen und weist eine Dicke von ca. 0,42 mm auf. Es wurde durch Extrusion hergestellt. Die Höhe der pilzförmigen Erhebung gegenüber dem Grund des Materials beträgt ca. 0,26 mm. Der Abstand der Kanten der Köpfe beträgt ca. 200 µm.

Weitere Merkmale, Einzelheiten und Vorteile der Erfindung ergeben sich aus den Patentansprüchen und aus der zeichnerischen Darstellung und nachfolgenden Beschreibung einer bevorzugten Ausführungsform der Erfindung. In der Zeichnung zeigt:
- Figur 1: eine schematische Ansicht eines erfindungsgemäßen Hygieneartikels mit einem Gürtel aus einem erfindungsgemäßen Vliesstoffmaterial;
- Figur 2: eine schematische Schnittansicht des Vliesstoffmaterials aus Figur 3a;
- Figur 3a: eine Draufsicht auf die Abwicklung einer Gravurwalze zum Prägen des erfindungsgemäßen Vliesstoffmaterials; außerdem gleichzeitig schematisch eine Ansicht der ersten Oberseite des Vliestoffmaterials;
- Figur 3b: eine Schnittansicht (Detail) aus Figur 3a;
- Figur 4: eine schematische Darstellung des Aufbaus eines Zugprüfversuchs mit einer Vorrichtung mit gekrümmter Fläche;
- Figur 5a-d: schematische Darstellungen des Prüflings;
- Figur 6: eine perspektivische Ansicht der in Figur 4 dargestellten Vorrichtung und
- Figuren 7 und 8: Darstellung einer hakenbildenden Komponente eines Verschlussmittels.

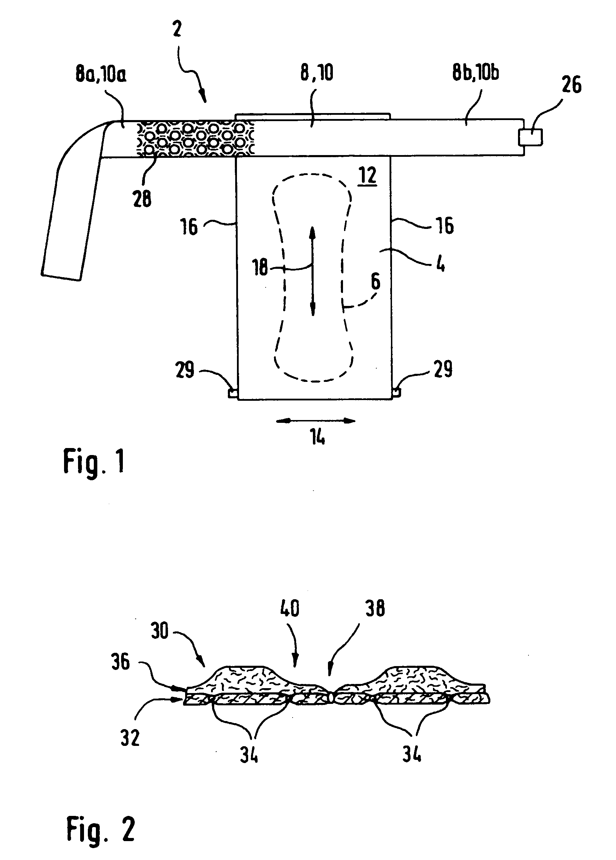

Figur 1 zeigt schematisch eine wegwerfbare Gürtelwindel 2 mit einem Hauptteil 4 und einem angedeuteten Absorptionskörper 6. An den Hauptteil 4 angefügt ist ein einstückiger Materialabschnitt 8, welcher einen Hüftgürtel 10 der Gürtelwindel bildet. Figur 1 zeigt den Gürtel in entfaltetem Zustand. Der einstückige Materialabschnitt 8 ist an eine Außenseite 12 des Hauptteils 4 unlösbar angefügt. Er erstreckt sich in Querrichtung 14 der Gürtelwindel 2 über seitliche Längsränder 16 im entfalteten Zustand jeweils über wenigstens 300 mm, insbesondere über wenigstens 400 mm, insbesondere über wenigstens 500 mm, insbesondere über wenigstens 600 mm, insbesondere über wenigstens 700 mm hinaus. Im dargestellten Fall der Figur 1 ist der erste Materialabschnitt 8a deutlich länger als der zweite Materialabschnitt 8b, das heißt die Erstreckung des ersten Hüftgürtelabschnittes 10a im entfalteten Zustand in Querrichtung 14 über den Längsrand 16 des Hauptteils 4 hinaus ist größer, vorzugsweise um 100mm größer, besonders bevorzugt um 200 mm größer ganz besonders bevorzugt um 300mm größer als die des zweiten Hüftgürtelabschnittes 10b.

Es wird aber darauf hingewiesen, dass erster und zweiter Materialabschnitt auch gleich lang sein können.

Am zweiten Hüftgürtelabschnitt 10b ist an dessen Ende auch ein Verschlussmittel 26 vorgesehen, und zwar in Form einer mechanische Verschlusselemente aufweisenden Lasche 26, welche mit Gegenverschlusselementen am ersten Hüftgürtelabschnitt 10a, insbesondere mit dessen gesamter Außenfläche lösbar haftend zusammenwirken kann, wenn der Hüftgürtel 10 zur Bildung einer in Hüftumfangsrichtung geschlossenen Hüftöffnung geschlossen wird. Vorteilhafterweise umfasst die Außenseite des Hüftgürtels ein erfindungsgemäßes schlaufenbildendes Vliesstoffmaterial, welches die Gegenverschlusselemente bildet. Dieses schlaufenbildende Vliesstoffmaterial ist mit Bezugszeichen 28 schematisch lediglich über eine begrenzte Länge des ersten Hüftgürtelabschnittes angedeutet. Nach einer bevorzugten Ausführungsform der Erfindung besteht der Hüftgürtel jedoch aus diesem Vliesstoffmaterial 28, so dass die schlaufenbildende Komponente des Vliesstoffmaterials die gesamte Außenseite des Hüftgürtels bildet.

Zum Anlegen der Gürtelwindel 2 wird der Hüftgürtel 10 auf sich selbst geschlossen und danach wird der Hauptteil 4 zwischen den Beinen eines Benutzers hervorgeholt und über weitere mechanische Verschlusselemente aufweisende Laschen 29 beidseits des Hauptteils 4 am Hüftgürtel 10 lösbar festgelegt.

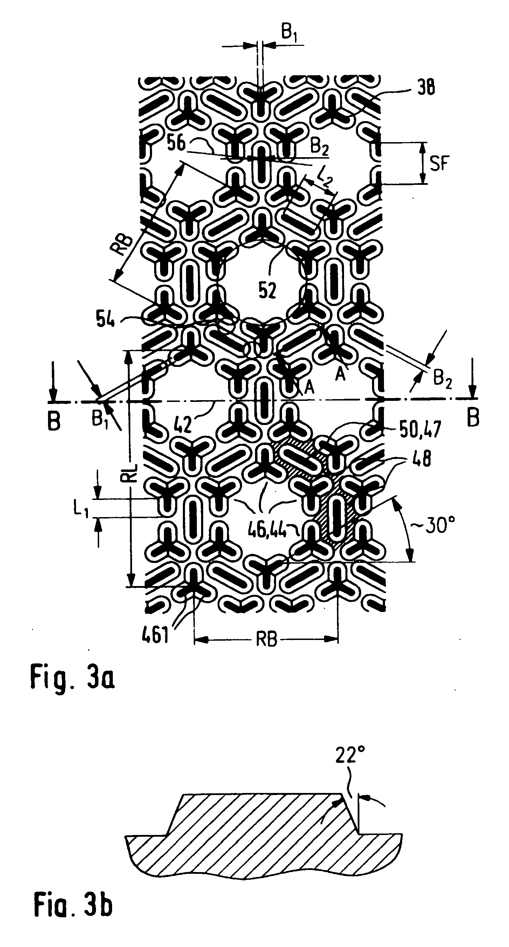

Das Vliesstoffmaterial 28 besteht aus einem Vliesstofflaminat 30, das im Schnitt in Figur 2 schematisch dargestellt ist. Die Schnittlinie verläuft dabei entlang einer aus Figur 3a ersichtlichen Linie B-B. Es weist eine Polypropylen - Spinnvliesschicht 32 bestehend aus Fasern einer Faserstärke von 2,2 dtex mit einem Flächengewicht von 30 g/m² als Träger auf, die durch ovale Bindepunkte 34 einer Flächendichte von 48,37 Stück/cm² (thermische Prägepunkte) vorverfestigt wurde, wobei die Abmessungen der Halbachsen der ovalen Bindepunkte jeweils 0,85 mm und 0,59 mm betragen und die Pressfläche der ovalen Bindepunkte dementsprechend 0,394 mm² und der Anteil der Bindepunkte an der Gesamtfläche somit 19,0 % beträgt. Die Tiefe der Bindepunkte beträgt 0,80 mm. Auf diese Spinnvliesschicht 32 ist eine Kardenvliesschicht 36 angefügt, und zwar durch eine Heißkalanderprägung. Im dargestellten Fall besteht das Kardenvlies aus hydrophilisierten Polypropylenfasern einer Stärke von 4,4 dtex und einer Faserlänge von 40 mm. Figur 3 zeigt eine Ansicht der Abwicklung der Oberfläche einer Kalanderwalze, welche einem durch Heißkalandern auf die erste Oberseite 40 der Kardenvliesschicht 36 aufgebrachten Prägemuster 38 entspricht Figur 3 zeigt schematisch somit gleichzeitig auch eine Ansicht der ersten Oberseite 40 des Vliesstofflaminats 30.

Durch Aufbringung des Prägemusters 38 werden auf der ersten Oberseite 40 des Vliesstofflaminats 30 erste größere ungebundene Bereiche 42 gebildet, die begrenzt werden durch eine thermisch gebundene Kontur 44 aus unterbrochenen ersten Segmenten 46. Diese ersten Segmente 46 weisen im dargestellten Fall eine in der Aufsicht regelmäßige trilobale Form auf. Die Arme 461 dieser ersten Segmente 46 weisen eine Länge L1 von 1,04 mm und eine Breite B1 von 0,47 mm auf. Die so begrenzten ersten ungebundenen Bereiche sind sechseckförmig und inselartig auf der ersten Oberseite 40 angeordnet und voneinander beabstandet. Zwischen den ersten Bereichen 42 befinden sich zweite ungebundene Bereiche 48, die kleiner als die ersten ungebundenen Bereiche 42 sind. Diese zweiten ungebundenen Bereiche umschließen wiederum gebundene Teilbereiche 50, die stegartig durch zweite Segmente 47 gebildet sind. Diese zweiten Segmente 47 weisen eine Länge L2 von 2,07 mm und eine Breite B2 von 0,47 mm auf. Die Prägetiefe der ersten und zweiten Segmente beträgt jeweils 0,59 mm. Im beispielhaft dargestellten Fall haben die ersten ungebundenen inselartigen Bereiche eine Abmessung der Seiten der Sechseckform SF von 2,9 mm. Ihr Abstand beträgt ca. 2,6 mm.

Es ist insbesondere nicht möglich, einen in die ersten größeren Bereiche 42 einbeschriebenen größtmöglichen Kreis 52, der im dargestellten Fall einen Durchmesser von 4,8 mm besitzt, innerhalb der zweiten ungebundenen kleineren Bereiche 48 anzuordnen. Dort sind größtmögliche Kreise 54 angedeutet.

Die Rapportbreite (RB) des Prägemusters 38 beträgt 8 mm, die Rapportlänge RL beträgt 13,86 mm.

Der gesamte Flächenanteil gebundener Bereiche 44, 50 beträgt 20,7 %. Der Flächenanteil der ersten größeren ungebundenen inselartigen Bereiche (42) beträgt ca. 35,7 %, wobei zur Ermittlung der Fläche eines ersten ungebundenen inselartigen Bereiches die Größe der Fläche des eingeschriebenen Kreises 52 herangezogen wird.

Die nach oben beschriebener Prüfmethode ermittelte Scherkraft dieses Vliesstoffmaterials beträgt 51,00 N/25 mm, wobei als Kletthakenkomponente das oben näher gekennzeichnete Material "Microplast" 42-288-HX200-PP3 von der Firma G. Binder GmbH & Co. KG Textil- und Kunststofftechnik, Holzgerlingen, verwendet wurde.

Die Steifigkeit des Vliesstoffmaterials ermittelt nach der in EP0699066B1 offenbarten Prüfmethode beträgt 0,13 N, wobei bei der Prüfanordnung die Spinnvliesseite oben zu liegen kommt, das heißt diejenige Oberfläche bildet, die dem Stempel der Prüfapparatur zugewandt ist.

Bei einem weiteren Ausführungsbeispiel der vorliegenden Erfindung wurde lediglich das Flächengewicht des Spinnvlieses auf 45 g/m² erhöht und das die Vliesstoffe verbindende Prägemuster 38 wie folgt modifiziert:
Länge L1 der Arme 461 der ersten Segmente 46: 1,0 mm
Breite B1 der Arme 461 der ersten Segmente 46: 0,4 mm
Länge L2 der zweiten Segmente 47: 2,0 mm
Breite B2 der zweiten Segmente 47: 0,4 mm
Flächenanteil gebundener Bereiche: 17,1 %
Prägetiefe der ersten und zweiten Segmente: 0,68 mm

Die nach oben beschriebener Prüfmethode ermittelte Scherkraft dieses weiteren Ausführungsbeispiels beträgt 58,89 N/25 mm, wobei als Kletthakenkomponente wiederum das oben näher gekennzeichnete Material "Microplast" 42-288-HX200-PP3 von der Firma G. Binder GmbH & Co. KG Textil- und Kunststofftechnik, Holzgerlingen, verwendet wurde.

Die Steifigkeit dieses weiteren Vliesstoffmaterials ermittelt nach der in EP0699066B1 offenbarten Prüfmethode beträgt 0,28 N, wobei bei der Prüfanordnung die Spinnvliesseite oben zu liegen kommt, das heißt diejenige Oberfläche bildet, die dem Stempel der Prüfapparatur zugewandt ist.

Jede gerade Verbindungslinie 56 zwischen benachbarten ersten größeren Bereichen 42 verläuft stets durch eine gebundene Kontur 44 oder einen gebundenen Teilbereich 50, der zwischen den ersten größeren Bereichen 42 angeordnet ist.

Bevorzugt und beispielhaft dargestellte Abmessungen der Prägestruktur 38 sind in den Figuren 3a und 3b dargestellt, wobei Figur 3b einen Schnitt entlang der Linie A-A durch ein zweites Segment 56 darstellt.

## Patentansprüche

1. Schlaufenbildendes Vliesstoffmaterial (28) als mechanisches Verschlussmittel, insbesondere bei wegwerfbaren Hygieneartikeln (2), wie Windeln, Gürtelwindeln, Inkontinenzartikeln und -vorlagen, wobei eine erste Oberseite (40) des Vliesstoffmaterials (30) erste größere ungebundene Bereiche (42) umfasst, die voneinander beabstandet inselartig angeordnet sind, **dadurch gekennzeichnet, dass** die ersten größeren ungebundenen Bereiche (42) von gebundenen Konturen (44) begrenzt sind und außerhalb der Begrenzung von zweiten kleineren ungebundenen Bereichen (48) umgeben und durch diese zweiten kleineren ungebundenen Bereiche (48) voneinander beabstandet sind, wobei die ersten größeren ungebundenen inselartigen Bereiche (42) eine Abmessung von 2 - 15 mm und einen Flächenanteil von 10 - 50 % der Gesamtfläche der ersten Oberseite (40) aufweisen, und wobei der gesamte Flächenanteil gebundener Bereiche 10 - 40 % der Gesamtfläche der ersten Oberseite (40) beträgt.

2. Vliesstoffmaterial nach Anspruch 1, **dadurch gekennzeichnet, dass** die zweiten kleineren ungebundenen Bereiche (48) ihrerseits gebundene Teilbereiche (50) aufweisen, insbesondere umschließen können.

3. Vliesstoffmaterial nach Anspruch 1 oder 2, **dadurch gekennzeichnet, dass** jede gerade Verbindungslinie (56) zwischen benachbarten ersten größeren Bereichen (42) stets durch eine gebundene Kontur (44) oder einen gebundenen Teilbereich (50), der zwischen den ersten größeren Bereichen (42) angeordnet ist, verläuft.

4. Vliesstoffmaterial nach einem oder mehreren der vorstehenden Ansprüche, **dadurch gekennzeichnet, dass** die gebundenen Konturen (44) Prägelinien oder Prägelinienabschnitte einer Breite von 0,2 - 1 mm, insbesondere von 0,2 - 0,8 mm und weiter insbesondere von 0,3 - 0,6 mm aufweisen.

5. Vliesstoffmaterial nach einem oder mehreren.der vorstehenden Ansprüche, **dadurch gekennzeichnet, dass** die gebundenen Konturen (44) Prägelinien oder Prägelinienabschnitte einer Tiefe von 0,4 - 1,5 mm, insbesondere von 0,4 - 0,7 mm, insbesondere von 0,5 - 0,7 mm aufweisen.

6. Vliesstoffmaterial nach einem oder mehreren der vorstehenden Ansprüche, **dadurch gekennzeichnet, dass** die ersten größeren ungebundenen inselartigen Bereiche (42) eine Abmessung, insbesondere einen Durchmesser eines in den ersten Bereich einbeschriebenen Kreises (52), von 3 - 10 mm und weiter insbesondere von 3 - 8 mm aufweisen.

7. Vliesstoffmaterial nach einem oder mehreren der vorstehenden Ansprüche, **dadurch gekennzeichnet, dass** die ersten größeren ungebundenen inselartigen Bereiche (42) einen Flächenanteil von 10 - 45 %, insbesondere von 15 - 45 %, insbesondere von 20 - 40 % und weiter insbesondere von 30 - 40 % der Gesamtfläche der ersten Oberseite aufweisen.

8. Vliesstoffmaterial nach einem oder mehreren der vorstehenden Ansprüche, **dadurch gekennzeichnet, dass** die ersten größeren ungebundenen inselartigen Bereiche (42) kreisförmig oder oval oder dreieckig oder vieleckig, insbesondere sechseckig, ausgebildet sind.

9. Vliesstoffmaterial nach einem oder mehreren der vorstehenden Ansprüche, **dadurch gekennzeichnet, dass** die ersten größeren ungebundenen inselartigen Bereiche (42) wenigstens 1 mm, insbesondere wenigstens 1,5 mm, insbesondere wenigstens 2 mm, insbesondere wenigstens 2,5 mm und weiter insbesondere wenigstens 3 mm voneinander beabstandet sind.

10. Vliesstoffmaterial nach einem oder mehreren der vorstehenden Ansprüche, **dadurch gekennzeichnet, dass** die ersten größeren ungebundenen inselartigen Bereiche (42) höchstens 10 mm, insbesondere höchstens 5 mm voneinander beabstandet sind.

11. Vliesstoffmaterial nach einem oder mehreren der vorstehenden Ansprüche, **dadurch gekennzeichnet, dass** der gesamte Flächenanteil gebundener Bereiche 15 - 40 %, insbesondere 15 - 30 %, insbesondere 15 - 25 %, insbesondere 19 - 22 % der Gesamtfläche der ersten Oberfläche beträgt.

12. Vliesstoffmaterial nach einem oder mehreren der vorstehenden Ansprüche, **dadurch gekennzeichnet, dass** sie ein Spinnvlies (32) oder ein Kardenvlies (36) oder ein Meltblownvlies oder ein wasservernadeltes Vlies umfasst.

13. Vliesstoffmaterial nach einem oder mehreren der vorstehenden Ansprüche, **dadurch gekennzeichnet, dass** sie ein Vlieslaminat (30) umfasst.

14. Vliesstoffmaterial nach Anspruch 13, **dadurch gekennzeichnet, dass** das Vlieslaminat (30) ein Spinnvlies (32) und ein Kardenvlies (36) umfasst, die miteinander insbesondere durch Thermoprägung, insbesondere durch Heißkalanderprägung verbunden sind.

15. Vliesstoffmaterial nach Anspruch 13 oder 14, **dadurch gekennzeichnet, dass** ein Kardenvlies (36) die erste Oberseite (40) bildet, welche eine Landezone für eine hakenbildende Komponente eines mechanischen Verschlussmittels bildet.

16. Vliesstoffmaterial nach einem oder mehreren der vorstehenden Ansprüche, **dadurch gekennzeichnet, dass** durch Anbringung der die ersten größeren ungebundenen Bereiche (42) begrenzenden gebundenen Konturen (44) Schichten des Vlieslaminats (30) miteinander verbunden werden.

17. Vliesstoffmaterial nach einem oder mehreren der vorstehenden Ansprüche, **dadurch gekennzeichnet, dass** eine von der ersten Oberseite (40) abgewandte Vliesschicht durch ein weiteres Bindemuster (34) verfestigt ist.

18. Vliesstoffmaterial nach einem oder mehreren der vorstehenden Ansprüche, **dadurch gekennzeichnet, dass** sie ein Flächengewicht von 15 bis 120 g/m², insbesondere von 20 bis 90 g/m², insbesondere von 30 bis 80 g/m², insbesondere von 40 bis 70 g/m² und weiter insbesondere von 50 bis 65 g/m² aufweist.

19. Vliesstoffmaterial nach einem oder mehreren der vorstehenden Ansprüche, **dadurch gekennzeichnet, dass** diejenige Vliesschicht, welche die erste Oberseite (40) bildet und die ersten ungebundenen Bereiche (42), die gebundenen Konturen (44) und die zweiten ungebundenen Bereiche (48) aufweist, ein Flächengewicht von 10 bis 60 g/m², insbesondere von 10 bis 40 g/m², insbesondere von 15 bis 35 g/m² und weiter insbesondere von 20 bis 35 g/m² aufweist.

20. Vliesstoffmaterial nach einem oder mehreren der vorstehenden Ansprüche, **dadurch gekennzeichnet, dass** diejenige Vliesschicht (36), welche die erste Oberseite (40) bildet, Fasern einer Stärke von 1 bis 10 dtex, insbesondere von 2 bis 8 dtex und weiter insbesondere von 3 bis 6 dtex umfasst oder aus solchen Fasern besteht.

21. Vliesstoffmaterial nach einem oder mehreren der vorstehenden Ansprüche, **dadurch gekennzeichnet, dass** diejenige Vliesschicht (36), welche die erste Oberseite (40) bildet, hydrophile Fasern umfasst oder aus hydrophilen Fasern besteht.

22. Vliesstoffmaterial nach einem oder mehreren der vorstehenden Ansprüche, **gekennzeichnet durch** eine weitere Vliesschicht (32) als Träger, die ein Flächengewicht von 10 bis 100 g/m², insbesondere von 15 bis 60 g/m² und weiter insbesondere von 30 bis 40 g/m² aufweist.

23. Vliesstoffmaterial nach Anspruch 22, **dadurch gekennzeichnet, dass** die weitere Vliesschicht (32) Fasern einer Stärke von 1 bis 6 dtex, insbesondere von 1 bis 4 dtex und weiter insbesondere von 2 bis 4 dtex umfasst oder aus solchen Fasern besteht.

24. Vliesstoffmaterial nach einem oder mehreren der vorstehenden Ansprüche, **dadurch gekennzeichnet, dass** sie eine Steifigkeit von < 0,80 N, insbesondere von < 0,60. N, insbesondere von < 0,40 N, insbesondere von < 0,30 N, insbesondere von < 0,25 N, insbesondere von < 0,20 N, insbesondere < 0,18 N, und weiter insbesondere < 0,16 N, jedoch von wenigstens 0,05 N aufweist.

25. Hygieneartikel, insbesondere Windel, Gürtelwindel, Inkontinenzartikel oder Inkontinenzvorlage, **gekennzeichnet durch** ein mechanisches Verschlussmittel, welches ein schlaufenbildendes Vliesstoffmaterial (28) nach einem oder mehreren der vorstehenden Ansprüche umfasst.

26. Hygieneartikel nach Anspruch 25, **gekennzeichnet durch** einen in Hüftumfangsrichtung eines Benutzers erstreckten und auf sich selbst schließbaren Gürtel (10).

27. Hygieneartikel nach Anspruch 26, **dadurch gekennzeichnet, dass** der Gürtel (10) von einem insbesondere einstückigen Materialabschnitt (8) gebildet ist, welcher Materialabschnitt (8) ein Vliesstoffmaterial (28) nach einem oder mehreren der Ansprüche 1 bis 24 umfasst oder hieraus gebildet ist.

28. Hygieneartikel nach einem der Ansprüche 25 bis 27 , **dadurch gekennzeichnet, dass** der Gürtel (10) oder das Vliesstoffmaterial (28) eine Erstreckung in Hüftumfangsrichtung oder Querrichtung (14) des Hygieneartikels von 25 bis 200 cm, insbesondere von 40 bis 180 cm, insbesondere von 60 bis 180 cm, insbesondere von 100 bis 180 cm und weiter von 120 bis 180 cm aufweist.

29. Hygieneartikel nach Anspruch 25 bis 28, **dadurch gekennzeichnet, dass** der Gürtel (10) oder das Vliesstoffmaterial (28) eine Erstreckung in Längsrichtung (18) des Hygieneartikels von 3 bis 20 cm, insbesondere von 3 bis 15 cm, insbesondere von 4 bis 10 cm und weiter insbesondere von 4 bis 6 cm aufweist.

30. Hygieneartikel nach einem der vorstehenden Ansprüche 25 bis 29, **dadurch gekennzeichnet, dass** das schlaufenbildende Vliesstoffmaterial (28) mit einer hakenbildenden Komponente eines mechanischen Verschlussmittels zum Schließen des Hygieneartikels derart zusammenwirkt, dass eine Haftkraft als Scherkraft von wenigstens 5 N/25 mm, insbesondere von wenigstens 10 N/25 mm, insbesondere von wenigstens 15 N/25 mm, insbesondere von wenigstens 20 N/25 mm, insbesondere von wenigstens 25 N/25 mm, insbesondere von wenigstens 30 N/25 mm, insbesondere von wenigstens 35 N/25 mm, insbesondere von wenigstens 40 N/25 mm, insbesondere von wenigstens 45 N/25 mm, insbesondere von wenigstens 50 N/25 mm, jedoch insbesondere von höchstens 80 N/25 mm und weiter insbesondere von höchstens 70 N/25 mm erreicht wird.

31. Mechanisch wirkendes Verschlussmittel mit einer schlaufenbildenden Komponente in Form eines Vliesstoffmaterials (28) nach einem oder mehreren der Ansprüche 1-24 und mit einer hakenbildenden Komponente.

32. Mechanisch wirkendes Verschlussmittel nach Anspruch 31, **gekennzeichnet durch** eine Haftkraft bei Scherbeanspruchung von wenigstens 5 N/25 mm, insbesondere von wenigstens 10 N/25 mm, insbesondere von wenigstens 15 N/25 mm, insbesondere von wenigstens 20 N/25 mm, insbesondere von wenigstens 25 N/25 mm, insbesondere von wenigstens 30 N/25 mm, insbesondere von wenigstens 35 N/25 mm, insbesondere von wenigstens 40 N/25 mm, insbesondere von wenigstens 45 N/25 mm, insbesondere von wenigstens 50 N/25 mm, jedoch insbesondere von höchstens 80 N/25 mm und weiter insbesondere von höchstens 70 N/25 mm.

## Claims

1. Loop-forming nonwoven material (28) as mechanical closure element, in particular, for disposable hygiene articles (2) such as diapers, belt diapers, incontinence articles and pads, wherein a first upper side (40) of the non-woven material (30) comprises first larger nonbonded areas (42) which are spaced apart from each other and disposed like islands, **characterized in that** the first larger nonbonded areas (42) are delimited by bonded contours (44) and are surrounded outside of the limitation by second smaller nonbonded areas (48) and are separated from each other by these second smaller nonbonded areas (48) and wherein the first larger non-bonded island-type areas (42) have a dimension of 2 - 15 mm and a surface portion of 10 to 50 % of the overall surface of the first upper side (40) and wherein the overall surface portion of bonded areas is 10 to 40 % of the overall area of the first upper side (40).

2. Nonwoven material according to claim 1, **characterized in that** the second smaller nonbonded areas (48) in turn have bonded partial areas (50) which they can, in particular, surround.

3. Nonwoven material according to claim 1 or 2, **characterized in that** each straight connecting line (56) between neighboring first larger areas (42) always extends through a bonded contour (44) or a bonded partial area (50) which is disposed between the first larger areas (42).

4. Nonwoven material according to any one or more of the preceding claims, **characterized in that** the bonded contours (44) have embossing lines or embossing line sections of a width of 0.2 to 1 mm, in particular 0.2 to 0.8 mm and, in particular 0.3 to 0.6 mm.

5. Nonwoven material according to any one or more of the preceding claims, **characterized in that** the bonded contours (44) have embossing lines or embossing line sections of a depth of 0.4 to 1.5 mm, in particular 0.4 to 0.7 mm, in particular 0.5 to 0.7 mm.

6. Nonwoven material according to any one or more of the preceding claims, **characterized in that** the first larger non-bonded island-type areas (42) have a dimension, in particular a diameter of a circle (52) inscribed into the first area of 3 to 10 mm, and in particular 3 to 8 mm.

7. Nonwoven material according to any one or more of the preceding claims, **characterized in that** the first larger non-bonded island-type areas (42) have a surface portion of 10 to 45 %, in particular 15 to 45 %, in particular 20 to 40 %, and in particular 30 to 40 % of the overall surface of the first upper side.

8. Nonwoven material according to any one or more of the preceding claims, **characterized in that** the first larger non-bonded island-type areas (42) have a circular, oval, triangular or polygonal, in particular hexagonal shape.

9. Nonwoven material according to any one or more of the preceding claims, **characterized in that** the first larger nonbonded island-type areas (42) have a mutual separation from each other of at least 1 mm, in particular at least 1.5 mm, in particular at least 2 mm, in particular at least 2.5 mm and in particular at least 3 mm.

10. Nonwoven material according to any one or more of the preceding claims, **characterized in that** the first larger nonbonded island-type areas (42) have a maximum mutual separation of maximally 10 mm, in particular maximally 5 mm.

11. Nonwoven material according to any one or more of the preceding claims, **characterized in that** the overall surface portion of bonded areas is 15 to 40 %, in particular 15 to 30 %, in particular 15 to 25 % and in particular 19 to 22 % of the overall area of the first upper side.

12. Nonwoven material according to any one or more of the preceding claims, **characterized in that** it comprises a spunbonded material (32) or a carded web (36) or a meltblown nonwoven or a water-needled nonwoven material.

13. Nonwoven material according to any one or more of the preceding claims, **characterized in that** it comprises a nonwoven laminate (30).

14. Nonwoven material according to claim 13, **characterized in that** the nonwoven laminate (30) comprises a spunbonded material (32) and a carded web (36) which are connected to each other, in particular, through thermo embossing, in particular through hot calendar embossing.

15. Nonwoven material according to claim 13 or 14, **characterized in that** a carded web (36) forms the first upper side (40) which forms a landing zone for a hook-forming component of a mechanical closure element.

16. Nonwoven material according to any one or more of the preceding claims, **characterized in that** layers of the non-woven laminate (30) are connected to each other through mounting the bonded contours (4) limiting the first larger nonbonded areas (42).

17. Nonwoven material according to any one or more of the preceding claims, **characterized in that** one nonwoven layer facing away from the first upper side (40) is solidified by a further bonding pattern (34).

18. Nonwoven material according to any one or more of the preceding claims, **characterized in that** it has a surface density of 15 to 120 g/m², in particular of 20 to 90 g/m², in particular of 30 to 80 g/m², in particular of 40 to 70 g/m², and in particular of 50 to 65 g/m².

19. Nonwoven material according to any one or more of the preceding claims, **characterized in that** the basis weight of that nonwoven layer which forms the first upper side (40) and has the first nonbonded areas (42), bonded contours (44) and second non-bonded areas (48) is 10 to 60 g/m², in particular 10 to 40 g/m², in particular 15 to 35 g/m², and moreover in particular 20 to 35 g/m².

20. Nonwoven material according to any one or more of the preceding claims, **characterized in that** the nonwoven layer (36) which forms the first upper side (40) has, or consists of fibers of a thickness of 1 to 10 dtex, in particular 2 to 8 dtex and in particular 3 to 6 dtex.

21. Nonwoven material according to any one or more of the preceding claims, **characterized in that** the nonwoven layer (36), which forms the first upper side (40), comprises hydrophilic fibers or consists of hydrophilic fibers.

22. Nonwoven material according to any one or more of the preceding claims, **characterized by** a further nonwoven layer (32) as a carrier which has a basis weight of 10 to 100 g/m², in particular 15 to 60 g/m², and in particular 30 to 40 g/m².

23. Nonwoven material according to claim 22, **characterized in that** the further nonwoven layer (32) has or consists of fibers of a thickness of 1 to 6 dtex, in particular of 1 to 4 dtex and in particular of 2 to 4 dtex.

24. Nonwoven material according to any one or more of the preceding claims, **characterized in that** it has a stiffness of < 0.80 N, in particular < 0.60 N, in particular < 0.40 N, in particular < 0.30 N, in particular < 0.25 N, in particular < 0.20 N, in particular < 0.18 N and moreover in particular < 0.16 N, however at least 0.05 N.

25. Hygiene article, in particular, different diapers, belt diapers incontinence article or pad, **characterized by** a mechanical closure element which comprises a loop-forming nonwoven material (28) in accordance with one or more of the preceding claims.

26. Hygiene article according to claim 25, **characterized by** a belt (10) that extends in the peripheral direction of the hip of a user and can be closed on top of itself.

27. Hygiene article according to claim 26, **characterized in that** the belt (10) is formed by an, in particular, one-piece material section (8) which comprises or is formed of a nonwoven material (28) in accordance with one or more of the claims 1 through 24.

28. Hygiene article according to any one of the claims 25 through 27, **characterized in that** the belt (10) or the nonwoven material (28) extends in the peripheral hip or transverse direction (14) of the hygiene article by 25 to 200 cm, in particular, 40 to 180 cm, in particular 60 to 180 cm, in particular 100 to 180 cm and moreover 120 to 180 cm.

29. Hygiene article according to claims 25 through 28, **characterized in that** the belt (10) or the nonwoven material (28) extends in the longitudinal direction (18) of the hygiene article by 3 to 20 cm, in particular 3 to 15 cm, in particular 4 to 10 cm and moreover in particular 4 to 6 cm.

30. Hygiene article according to any one of the preceding claims 25 through 29, **characterized in that** the loop-forming nonwoven material (28) cooperates with a hook-forming component of a mechanical closure element for closing the hygiene article, such that an adherent force as a shearing force of at least 5 N/25 mm, in particular at least 10 N/25 mm in particular at least 15N/25mm, in particular at least 20N/25mm, in particular at least 25N/25mm, in particular at least 30 N/25mm, in particular at least 35 N/25 mm, in particular at least 40 N/25 mm, in particular at least 45 N/25 mm, in particular at least 50 N/25 mm, however in particular of at most 80 N/25mm and moreover in particular at most 70 N/25 mm is achieved.

31. Mechanically acting closure element with a loop-forming component in the form of a non-woven material (28) in accordance with one or more of the claims 1 through 24, and with a hook-forming component.

32. Mechanically acting closure element according to claim 31, **characterized by** an adherent force at a shearing stress of at least 5 N/25 mm, in particular at least 10 N/25 mm in particular at least 15 N/25 mm, in particular at least 20 N/25 mm, in particular at least 25 N/25 mm, in particular at least 30 N/25 mm, in particular at least 35 N/25 mm, in particular at least 40 N/25 mm, in particular at least 45 N/25 mm, in particular at least 50 N/25 mm, however, in particular at most 80 N/25mm and moreover at most 70 N/25 mm.

## Revendications

1. Matière non tissée formant des boucles (28) en tant qu'élément d'obturation mécanique, en particulier pour des articles hygiéniques jetables (2), tels que des couches, des couches-culottes, des articles et des protections contre l'incontinence, une première face supérieure (40) de la matière non tissée (30) comprenant des premières zones étendues non liées (42) espacées les unes des autres à la façon d'ilots, **caractérisée en ce que** les premières zones étendues non liées (42) sont limitées par des contours liés (44) et, au-delà de cette limite, entourées par des secondes zones restreintes non liées (48) et espacées les unes des autres par ces secondes zones restreintes non liées (48), les premières zones étendues non liées du type ilot (42) présentant une dimension atteignant 2-15 mm et une proportion superficielle de 10-50 % de la surface totale de la première face supérieure (40), et la proportion superficielle totale des zones liées atteignant 10-40% de la surface totale de la première face supérieure (40).

2. Matière non tissée selon la revendication 1, **caractérisée en ce que** les secondes zones restreintes non liées (48) peuvent comprendre, en particulier entourer pour leur part des zones partielles liées (50).

3. Matière non tissée selon la revendication 1 ou 2, **caractérisée en ce que** chaque ligne de liaison rectiligne (56) s'étend entre des premières zones étendues (42) voisines à chaque fois à travers un contour lié (44) ou une zone partielle liée (50) disposée entre les premières zones étendues (42).

4. Matière non tissée selon l'une ou plusieurs des revendications précédentes, **caractérisée en ce que** les contours liés (44) présentent des lignes d'estampage ou des segments de ligne d'estampage d'une largeur de 0,2-1 mm, en particulier de 0,2-0,8 mm et plus particulièrement de 0,3-0,6 mm.

5. Matière non tissée selon l'une ou plusieurs des revendications précédentes, **caractérisée en ce que** les contours liés (44) présentent des lignes d'estampage ou des segments de ligne d'estampage d'une profondeur de 0,4-1,5 mm, en particulier de 0,4-0,7 mm, en particulier de 0,5-0,7 mm.

6. Matière non tissée selon l'une ou plusieurs des revendications précédentes, **caractérisée en ce que** les premières zones étendues non liées du type ilot (42) présentent une dimension, en particulier un diamètre d'un cercle (52) inscrit dans la première zone, de 3-10 mm et plus particulièrement de 3-8 mm.

7. Matière non tissée selon l'une ou plusieurs des revendications précédentes, **caractérisée en ce que** les premières zones étendues non liées du type ilot (42) présentent une proportion superficielle de 10-45 %, en particulier de 15-45 %, en particulier de 20-45 % et plus particulièrement de 30-40 % % de la surface totale de la première face supérieure.

8. Matière non tissée selon l'une ou plusieurs des revendications précédentes, **caractérisée en ce que** les premières zones étendues non liées de type ilot (42) présentent une forme circulaire ou ovale ou triangulaire ou polygonale, en particulier hexagonale.

9. Matière non tissée selon l'une ou plusieurs des revendications précédentes, **caractérisée en ce que** les premières zones étendues non liées du type ilot (42) sont espacées les unes des autres d'au moins 1 mm, en particulier d'au moins 1,5 mm, en particulier d'au moins 2 mm, en particulier d'au moins 2,5 mm et plus particulièrement d'au moins 3 mm.

10. Matière non tissée selon l'une ou plusieurs des revendications précédentes, **caractérisée en ce que** les premières zones étendues non liées du type ilot (42) sont espacées les unes des autres d'au maximum 10 mm, en particulier d'au maximum 5 mm.

11. Matière non tissée selon l'une ou plusieurs des revendications précédentes, **caractérisée en ce que** la proportion superficielle totale des zones liées atteint 15-40 %, en particulier 15-30 %, en particulier 15-25 %, en particulier 19-22 % de la surface totale de la première surface.

12. Matière non tissée selon l'une ou plusieurs des revendications précédentes, **caractérisée en ce qu'**elle comprend un filé-lié (32) ou un voile de carde (36) ou un non-tissé obtenu par fusion-soufflage ou un non-tissé aiguilleté à l'eau.

13. Matière non tissée selon l'une ou plusieurs des revendications précédentes, **caractérisée en ce qu'**elle comprend un stratifié non tissé (30).

14. Matière non tissée selon la revendication 13, **caractérisée en ce que** le stratifié non tissé (30) comprend un filé-lié (32) et un voile de carde (36) reliés ensemble en particulier par estampage à chaud, en particulier par estampage et calandrage à chaud.

15. Matière non tissée selon la revendication 13 ou 14, **caractérisée en ce qu'**un voile de carde (36) forme la première face supérieure (40) formant une zone d'atterrissage pour un composant formant des crochets d'un moyen d'obturation mécanique.

16. Matière non tissée selon l'une ou plusieurs des revendications précédentes, **caractérisée en ce que** les couches du stratifié non tissé (30) sont reliées ensemble par disposition des contours liés (44) limitant les premières zones étendues non liées (42).

17. Matière non tissée selon l'une ou plusieurs des revendications précédentes, **caractérisée en ce qu'**une couche de non-tissé opposée à la première face supérieure (40) est consolidée par un autre motif de soutien (34).

18. Matière non tissée selon l'une ou plusieurs des revendications précédentes, **caractérisée en ce qu'**elle présente un poids surfacique de 15 à 120 g/m², en particulier de 20 à 90 g/m², en particulier de 30 à 80 g/m², en particulier de 40 à 70 g/m² et plus particulièrement de 50 à 65 g/m².

19. Matière non tissée selon l'une ou plusieurs des revendications précédentes, **caractérisée en ce que** la couche de non-tissé formant la première face supérieure (40) et comprenant les premières zones non liées (42), les contours liés (44) et les secondes zones non liées (48) présente un poids surfacique de 10 à 60 g/m², en particulier de 10 à 40 g/m², en particulier de 15 à 35 g/m² et plus particulièrement de 20 à 35 g/m².

20. Matière non tissée selon l'une ou plusieurs des revendications précédentes, **caractérisée en ce que** la couche de non-tissé (36) formant la première face supérieure (40) comprend des fibres d'une épaisseur de 1 à 10 dtex, en particulier de 2 à 8 dtex et plus particulièrement de 3 à 6 dtex ou est constituée de telles fibres.

21. Matière non tissée selon l'une ou plusieurs des revendications précédentes, **caractérisée en ce que** la couche de non-tissé (36) formant la première face supérieure (40) comprend des fibres hydrophiles ou est constituée de fibres hydrophiles.

22. Matière non tissée selon l'une ou plusieurs des revendications précédentes, **caractérisée par** une autre couche de non-tissé (32) comme support, laquelle présente un poids surfacique de 10 à 100 g/m², en particulier de 15 à 60 g/m² et plus particulièrement de 30 à 40 g/m².

23. Matière non tissée selon la revendication 22, **caractérisée en ce que** l'autre couche de non-tissé (32) comprend des fibres d'une épaisseur de 1 à 6 dtex, en particulier de 1 à 4 dtex et plus particulièrement de 2 à 4 dtex ou est constituée de ces fibres.

24. Matière non tissée selon l'une ou plusieurs des revendications précédentes, **caractérisée en ce qu'**elle présente une rigidité < 0,80 N, en particulier < 0,60 N, en particulier < 0,40 N, en particulier < 0,30 N, en particulier < 0,25 N, en particulier < 0,20 N, en particulier < 0,18 N et plus particulièrement < 0,16 N, mais d'au moins 0,05 N.

25. Article hygiénique, en particulier couche pour bébé, couche-culotte, article contre l'incontinence ou protection contre l'incontinence, **caractérisé par** un moyen d'obturation mécanique comprenant une matière non tissée (28) formant des boucles selon l'une ou plusieurs des revendications précédentes.

26. Article hygiénique selon la revendication 25, **caractérisé par** une ceinture (10) s'étendant dans le sens circonférentiel des hanches d'un utilisateur et pouvant se fermer sur elle-même.

27. Article hygiénique selon la revendication 26, **caractérisé en ce que** la ceinture (10) est formée par un segment de matière (8) en particulier en une seule pièce, ledit segment de matière (8) comprenant une matière non tissée (28) selon l'une ou plusieurs des revendications 1 à 24, ou en étant constitué.

28. Article hygiénique selon l'une quelconque des revendications 25 à 27, **caractérisé en ce que** la ceinture (10) ou la matière non tissée (28) présente une étendue dans le sens circonférentiel des hanches ou le sens transversal (14) de l'article hygiénique de 25 à 200 cm, en particulier de 40 à 180 cm, en particulier de 60 à 180 cm, en particulier de 100 à 180 cm et plus particulièrement de 120 à 180 cm.

29. Article hygiénique selon la revendication 25 à 28, **caractérisé en ce que** la ceinture (10) ou la matière non tissée (28) présente une étendue dans le sens longitudinal (18) de l'article hygiénique de 3 à 20 cm, en particulier de 3 à 15 cm, en particulier de 4 à 10 cm et plus particulièrement de 4 à 6 cm.

30. Article hygiénique selon l'une quelconque des revendications 25 à 29, **caractérisé en ce que** la matière non tissée (28) formant des boucles coopère avec un composant formant des crochets d'un moyen d'obturation mécanique pour fermer l'article hygiénique, de telle sorte qu'une force adhésive comme force de cisaillement d'au moins 5 N/25 mm, en particulier d'au moins 10 N/25 mm, en particulier d'au moins 15 N/25 mm, en particulier d'au moins 20 N/25 mm, en particulier d'au moins 25 N/25 mm, en particulier d'au moins 30 N/25 mm, en particulier d'au moins 35 N/25 mm, en particulier d'au moins 40 N/25 mm, en particulier d'au moins 45 N/25 mm, en particulier d'au moins 50 N/25 mm, mais en particulier de maximum 80 N/25 mm et plus particulièrement de maximum 70 N/25 mm soit atteinte.

31. Moyen de fermeture agissant mécaniquement, doté d'un composant formant des boucles sous forme d'une matière non tissée (28) selon l'une ou plusieurs des revendications 1-24 et doté d'un composant formant des crochets.

32. Moyen de fermeture agissant mécaniquement selon la revendication 31, **caractérisé par** une force adhésive lors d'une sollicitation au cisaillement d'au moins 5 N/25 mm, en particulier d'au moins 10 N/25 mm, en particulier d'au moins 15 N/25 mm, en particulier d'au moins 20 N/25 mm, en particulier d'au moins 25 N/25 mm, en particulier d'au moins 30 N/25 mm, en particulier d'au moins 35 N/25 mm, en particulier d'au moins 40 N/25 mm, en particulier d'au moins 45 N/25 mm, en particulier d'au moins 50 N/25 mm, mais en particulier de maximum 80 N/25 mm et plus particulièrement de maximum 70 N/25 mm.
